(19) Europäisches Patentamt
European Patent Office
Office européen des brevets

(11) Veröffentlichungsnummer : **0 133 489**
**B1**

(12) # EUROPÄISCHE PATENTSCHRIFT

(45) Veröffentlichungstag der Patentschrift : **20.01.88**

(21) Anmeldenummer : **84108375.1**

(22) Anmeldetag : **17.07.84**

(51) Int. Cl.⁴ : **C 07 C 49/84**, C 07 C 43/225, C 07 C 39/42, C 07 C 69/75, C 07 C 25/13, C 09 K 19/30

(54) **Nematische Verbindungen.**

(30) Priorität : **04.08.83 CH 4234/83**
**23.03.84 DE 3410734**

(43) Veröffentlichungstag der Anmeldung : **27.02.85 Patentblatt 85/09**

(45) Bekanntmachung des Hinweises auf die Patenterteilung : **20.01.88 Patentblatt 88/03**

(84) Benannte Vertragsstaaten : **AT CH DE FR GB IT LI NL**

(56) Entgegenhaltungen :
**EP-A- 0 023 728**
**EP-A- 0 056 501**
**EP-A- 0 084 194**
**GB-A- 2 123 409**
**US-A- 4 393 258**
**Die Akte enthält technische Angaben, die nach dem Eingang der Anmeldung eingereicht wurden und die nicht in dieser Patentschrift enthalten sind.**

(73) Patentinhaber : **Merck Patent Gesellschaft mit beschränkter Haftung**
**Frankfurter Strasse 250**
**D-6100 Darmstadt (DE)**

(72) Erfinder : **Kelly, Stephen M., Dr.**
**Badenerstrasse 37**
**CH-5452 Oberrohrdorf (CH)**

**EP 0 133 489 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue nematische Verbindungen mit negativer DK-Anisotropie, die sich als Komponenten für Flüssigkristall (FK)-mischungen eignen. Es sind dies Verbindungen der Formel I

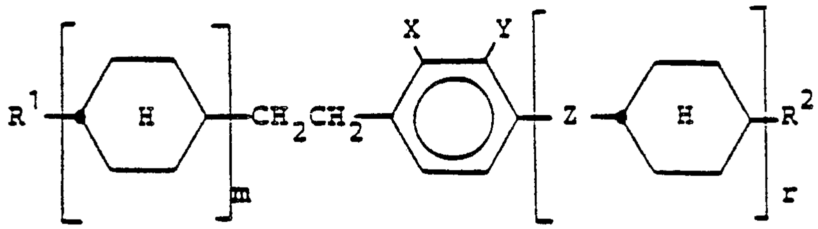

(I)

in welcher m = 1 oder 2, r = Null oder 1 und m + r = 1 oder 2 ist, und $R^1$ und $R^2$ gleich oder verschieden und gewählt sind aus Alkyl- und Alkoxygruppen mit jeweils 1-12 C-Atomen in gerader oder verzweigter und gegebenenfalls chiraler Kette, Z ein Brückenglied gewählt aus der Einfachbindung und den Gruppen der Formeln $-CH_2CH_2-$, $-OCH_2-$, —O—CO— oder —CO—O— ist, eine der Gruppen X, Y gewählt ist aus Fluor, Chlor, Brom und Cyano und die andere der Gruppen X, Y gewählt ist aus Wasserstoff, Fluor, Chlor, Brom und Cyano, mit der Maßgabe, daß m = r = 1 ist, wenn beide X, Y Cyano bedeuten.

Die Erfindung betrifft auch nematische FK-Mischungen, welche die neuen Verbindungen als Komponenten enthalten und eine insgesamt negative DK-Anisotropie besitzen.

Für den Betrieb bestimmter Anzeigen, z. B. für die sogenannten Guest/Host-Displays (GHD) und die homotropnematischen Displays (HND) mit Positivbildanzeige bzw. Positivkontrast, werden nematische Flüssigkristallmischungen mit negativer DK-Anisotrope (DKA) benötigt. Ähnliche Verbindungen sind bekannt, z. B. aus Dubois, J.C. et al. Mol. Cryst. Liq. Cryst. 42 (1977) 139 ; DE-OS Nrn. 22 40 864, 26 13 293, 28 35 662, 29 37 700, SU-PS 562'547 und EP Patentanmeldung Nr. 80200464.8.

Aus GB-A-2 123 409 und US-A-4 393 258 sind strukturell ähnliche Verbindungen ohne laterale Substitution (X = Y = H) bekannt.

Jedoch sind mit den bisher bekannten Verbindungen noch keine zufriedenstellenden nematischen FK-Mischungen erhältlich, die eine ausreichend stark negative DKA (z. B. 2 oder besser) in Kombination mit den für einen einwandfreien Betrieb von Anzeigestellen nötigen weiteren Eigenschaften von FK-Mischungen, wie allgemein-chemische, fotochemische, elektrochemische und thermische Stabilität, und einem ausreichend breiten Anisotropiebereich sowie ausreichend kurzen Ansprechzeiten auch bei niedrigen Temperaturen, z. B. höchstens etwa eine Sekunde am unteren Ende des Anisotropiebereichs, bieten.

Der Erfindung lag die Aufgabe zugrunde, neue nematische Verbindungen aufzufinden, welche die Herstellung von FK-Mischungen mit deutlich negativer DKA und den genannten weiteren Eigenschaften ermöglichen.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen mit insgesamt negativer optischer Anisotropie und vergleichsweise niedriger Viskosität herstellbar.

Die Verbindungen der Formel I können wie ähnliche Verbindungen als Komponenten flüssigkristalliner Phasen verwendet werden, wie sie besonders für elektrooptische Anzeigen nach Art von Guest/Host-Displays oder homotropnematischen Displays benötigt werden.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Gegenstand der Erfindung sind somit die Verbindungen der Formel I sowie die Verwendung der Verbindungen der Formel I als Komponenten flüssigkristalliner Mischungen. Gegenstand der Erfindung sind ferner flüssigkristalline Mischungen mit einem Gehalt an mindestens einer Verbindung der Formel I sowie elektrooptische Anzeigeelemente, die derartige Mischungen enthalten.

In diesen neuen Verbindungen liegen die Cyclohexylringe jeweils in trans-Konfiguration vor, wie durch Punkte in Formel I angedeutet.

Mindestens eine der Gruppen X, Y ist ein polarisierend wirkender Substituent aus der Gruppe Fluor, Chlor, Brom und Cyano.

Besonders bevorzugt sind Verbindungen der Formel I worin eine der Gruppen X, Y Fluor, Chlor oder Cyano ist, insbesondere solche, worin X Fluor bedeutet und Y Wasserstoff ist.

Wenn r = 1 ist, kann das dann vorhande Brückenglied Z eine Einfachbindung oder eine Gruppierung gewählt aus den Gruppen der Formeln $-CH_2CH_2-$, $-OCH_2-$, —O—CO— oder —CO—O— sein. Vorzugsweise ist Z $-CH_2CH_2-$, $-OCH_2-$ oder —O—CO—.

$R^1$ und $R^2$ sind gleiche oder verschiedene Flügelgruppen gewählt aus $C_1$-$C_{12}$-Alkyl- und $C_1$-$C_{12}$-Alkoxygruppen mit gerader oder verzweigter und gegebenenfalls chiraler Kette des Alkylteils, wie z. B. Methyl, Ethyl, Propyl, Butyl, Pentyl, Hexyl, Heptyl, Octyl, Nonyl, Decyl, Undecyl oder Dodecyl als Alkylteil

einschließlich der entsprechenden chiralisomeren Alkylteile, wie beispielsweise 1-Methylpropyl, 1-Methylbutyl, 2-Methylbutyl, 1-Methylpentyl, 2-Methylpentyl, 3-Methylpentyl, 1- oder 2- oder 3- oder 4-Methylhexyl, 1- oder 2- oder 3- oder 4- oder 5-Methylheptyl sowie weitere nach diesem Prinzip gebildete Alkyl-Alkylgruppen mit asymmetrischem C-Atom. Alkylteile mit 2-8 C-Atomen, insbesondere 3-8 C-Atomen, werden häufigbevorzugt.

$R^1$ ist vorzugsweise geradkettiges Alkyl mit 2-8 C-Atomen. $R^2$ ist im Falle $r = 0$ vorzugsweise geradkettiges Alkoxy mit 2-8 C-Atomen, im Falle $r = 1$ vorzugsweise geradkettiges Alkyl mit 2-8 C-Atomen.

Bevorzugte Gruppen erfindungsgemäßer Verbindungen der Formel I haben die folgenden Formeln Ia, Ib, Ic, Id, Ie und If, in welchen $R^1$, $R^2$, X und Y die oben für Formel I angegebene Bedeutung haben :

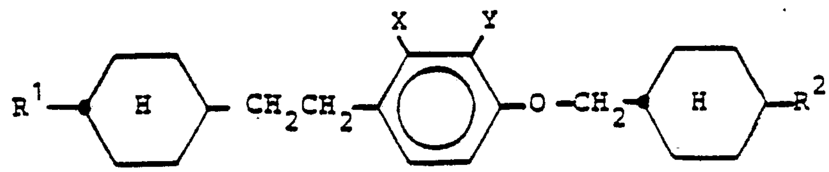

(Ia)

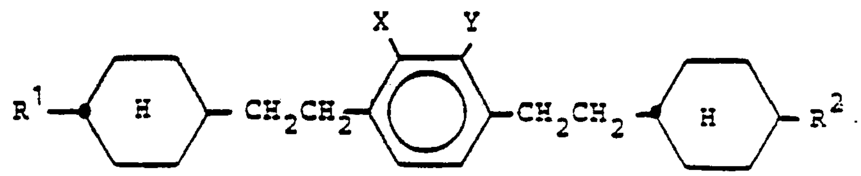

(Ib)

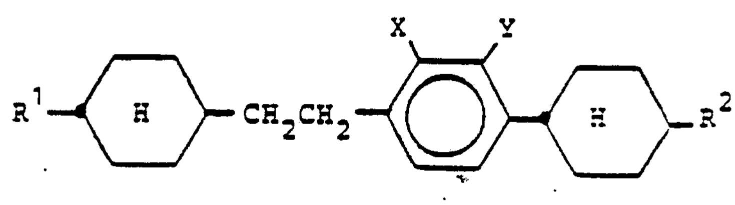

(Ic)

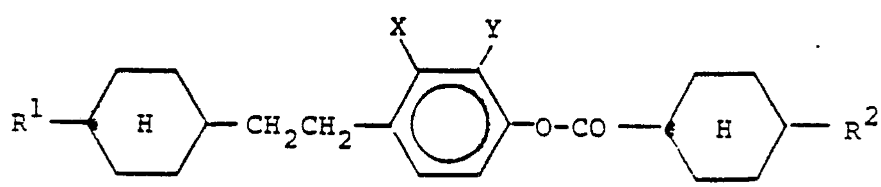

(Id)

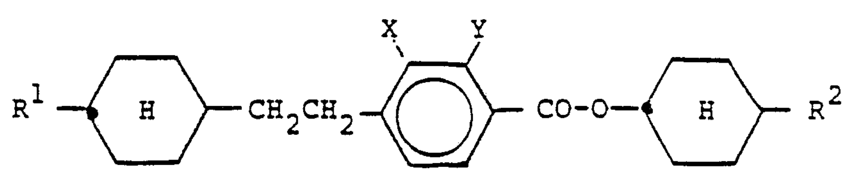

(Ie)

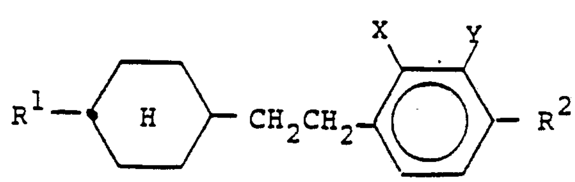

(If)

Bevorzugte Verbindungen der Formel If sind diejenigen, worin $R^2$ Alkoxy bedeutet und $R^1$ Alkyl ist.

Die neuen Verbindungen I können nach verschiedenen Verfahren hergestellt werden. Ein erstes allgemeines Verfahren beruht auf der Kondensation eines entsprechenden Carbonsäurederivates der Formel A—$CH_2COOH$, vorzugsweise in Form des entsprechenden Säurehalogenides, in welcher A den linken Molekülteil der Verbindung I, d. h.

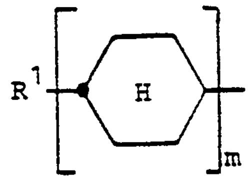

(A)

bedeutet, mit einem entsprechenden Anisolderivat der Formel B

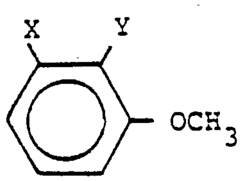

(B)

um das entsprechende Keton der Formel C

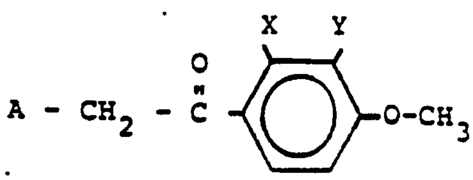

(C)

zu bilden, das dann zum Ersatz des Ketocarbonylsauerstoffes durch zwei Wasserstoffatome unter Bildung einer Verbindung der Formel D

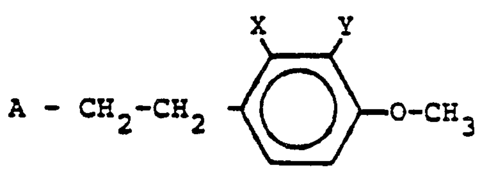

(D)

reduziert wird.

Zur Modifizierung von $R^2$ oder/und (wenn $r = 1$) zur Einführung der Struktur E

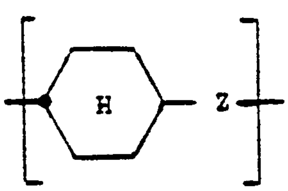

(E)

kann man die Verbindung D in eine Verbindung F

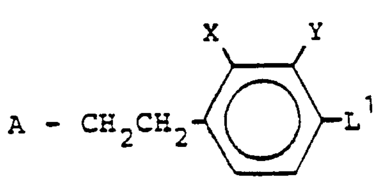

(F)

umwandeln, in der $L^1$ eine erste reaktive Gruppe, wie Hydroxyl, COOH oder Halogen bedeutet, und die Verbindung F nachfolgend mit einer Verbindung der Formel G

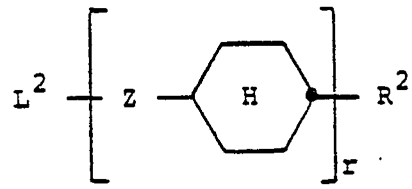

(G)

in der $L^2$ eine zweite reaktive Gruppe ist, zur Bildung der Zielverbindung I oder einer in diese umwandelbaren Vorverbindung umsetzen.

Alternativ können die Verbindungen I auch aus entsprechenden Vorverbindungen analoger Struktur aber ohne querpolarisierende Substituenten durch Halogenierung bzw. Transhalogenierung und gegebenenfalls Nitrilieren (Ersatz von Halogen durch Cyano) hergestellt werden.

Ein spezielles Beispiel für die Synthese bevorzugter Verbindungen I wird anhand des nachfolgenden Reaktionsschemas erläutert :

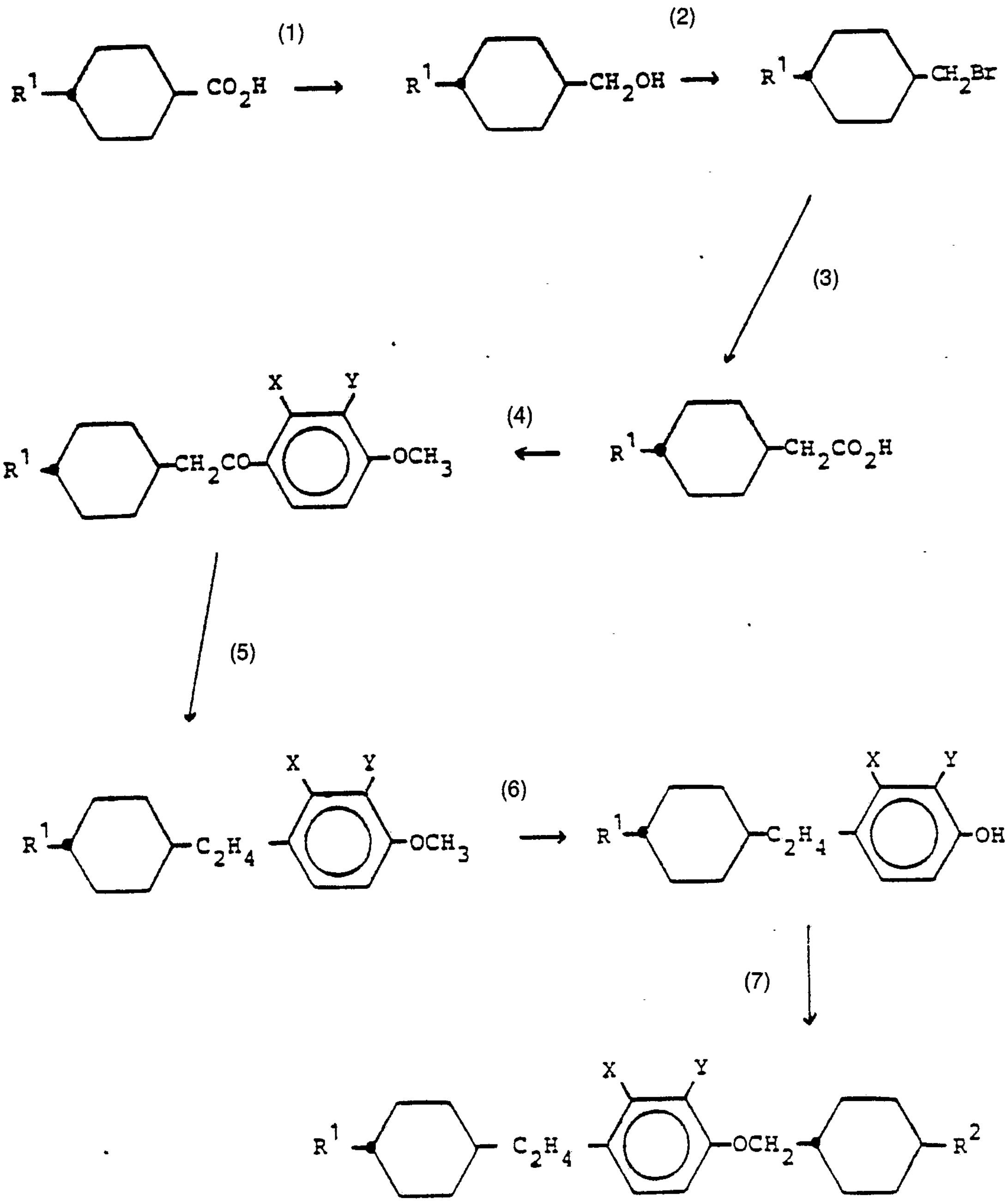

(1) Reduktion, z. B. mit $LiAlH_4$
(2) Bromierung, z. B. mit elementarem Brom in Acetonitril
(3) $CO_2$-Grignard
(4) Umwandlung in Säurehalogenid und Umsetzung mit 2—Y—3—X—Anisol
(5) Reduktion
(6) Demethylierung, z. B. mit Bortribromid
(7) Kondensation mit (trans-4-$R^2$-Cyclohexyl) methylbromid.

Die für die verschiedenen Synthesen der Verbindungen I benötigten Ausgangsstoffe sind entweder bekannt oder können nach an sich bekannten Methoden hergestellt werden.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind ; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Zur Herstellung erfindungsgemäßer FK-Mischungen wird jeweils mindestens eine spezielle Verbindung I als Komponente verwendet, gegebenenfalls mit anderen speziellen Verbindungen I oder/und mit für die Herstellung von FK-Mischungen mit klar negativer DKA zur Verwendung für GHD- oder HND-Systeme bekannten Verbindungen vermischt. Die mindestens eine Verbindung I macht dabei in der Regel mindestens 5 Mol% und maximal 30 Mol% der FK-Mischung aus. Bei Verwendung von mehreren Verbindungen I können diese bei entsprechender Auswahl einen überwiegenden Anteil, z. B. 50-90 Mol%, der Mischung darstellen.

FK-Mischungen, die praktisch nur aus Verbindungen I bestehen, gehören ebenfalls zur Erfindung.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent ; alle Temperaturen sind in Grad Celsius angegeben. « Übliche Aufarbeitung » bedeutet : man gibt Wasser hinzu, extrahiert mit Methylenchlorid, trennt ab, trocknet die organische Phase, dampft ein und reinigt das Produkt durch Kristallisation und/oder Chromatographie. Die angegebenen Phasenübergangstemperaturen beziehen sich auf Grad Celsius und stehen jeweils zwischen den entsprechenden Zeichen der Phasen « K » (kristallin), « N » (nematisch), « I » (isotrop), « $S_A$ » (A-smektisch).

## Beispiel 1

(a) Herstellung von trans-4-Pentylcyclohexylessigsäurechlorid

trans-4-Pentylcyclohexylessigsäure (12,0 g, 0,0566 Mol) wurde zur Umwandlung in das Säurechlorid auf einem Oelbad bei 80 °C etwa 1 Std. mit Thionylchlorid (50 ml) umgesetzt. Die erhaltene Lösung wurde unter vermindertem Druck eingedampft, mit absolutem Toluol (50 ml) versetzt und nochmals unter vermindertem Druck eingedampft.

(b) Herstellung von 2-Fluor-4-methoxy-α-(trans-4-pentyl-cyclohexyl) acetophenon

Das nach Absatz (a) erhaltene Säurechlorid wurde ohne weitere Reinigung in absolutem Dichlormethan (50 ml) aufgenommen und unter praktisch wasserfreien Bedingungen zu einer Lösung von 3-Fluoranisol (7,1 g, 0,0566 Mol), Aluminiumchlorid (8,3 g, 0,0623 Mol) und absolutem Dichlormethan (100 ml) zugetropft. Die so entstandene Mischung wurde 12 Std. gerührt und dann auf Eiswasser gegossen, das 50 ml konzentrierte Salzsäure enthielt. Die organische Phase wurde abgetrennt und die wässrige Phase mit Dichlormethan (2 × 100 ml) nachextrahiert. Die vereinigten organischen Phasen wurden in der Folge mit Wasser, Natriumbicarbonat und Wasser gewaschen und über Magnesiumsulfat getrocknet. Das nach Abdampfen des Dichlormethans unter vermindertem Druck erhaltene rohe Produkt wurde durch Umkristallisation aus Hexan bei 0 °C gereinigt. Dies ergab 11,5 g (64 % Ausbeute) reines 2-Fluor-4-methoxy-α-(trans-4-pentylcyclohexyl) acetophenon, F 71 bis 72 °C.

(c) Herstellung von 2-(trans-4-Pentylcyclohexyl)-1-(2-fluor-4-methoxyphenyl) ethan

Eine Mischung aus dem nach Absatz (b) erhaltenen 2-Fluor-4-methoxy-α-(trans-4-pentylcyclohexyl) acetophenon (10,0 g, 0,0313 Mol), Aluminiumlithiumhydrid (2,1 g, 0,0560 Mol), Aluminiumchlorid (15,0 g, 0,1125 Mol), absoluten Aether (70 ml) und absolutem Chloroform (70 ml) wurde 5,5 Std. bei praktisch wasserfreien Bedingungen auf schwachen Rückfluss erhitzt.

Die Reaktionslösung wurde dann abgekühlt und in der Folge mit wasserhaltigem Aether, Wasser und konzentrierter Salzsäure behandelt. Die wässrige Phase wurde abgetrennt und noch zweimal mit Aether (2 × 100 ml) extrahiert. Die vereinigten organischen Phasen wurden in der Folge mit Wasser, Natriumbicarbonat und Wasser gewaschen, über Magnesiumsulfat getrocknet und dann eingeengt. Das Rohprodukt wurde im Kugelrohr bei 150 °C/ca. $4 \times 10^{-2}$ Torr destilliert. Dieses Produkt (9,0 g, 94 % Ausbeute) kann ohne weitere Reinigung für die folgende Reaktion verwendet werden.

(d) Herstellung von 3-Fluor-4-[2-(trans-4-pentylcyclohexyl)-1-ethyl] phenol

Eine Lösung des nach Absatz (c) gewonnenen 2-(trans-4-Pentylcyclohexyl)-1-(2-fluor-4-methoxyphenyl) ethans (9,0 g, 0,0294 Mol) in absolutem Dichlormethan (100 ml) wurde tropfenweise zu einer Lösung von Bortribromid (11,0 g, 0,0441 Mol) und absolutem Dichlormethan (100 ml) unter praktisch wasserfreien Bedingungen bei 0 °C gegeben. Die so entstandene Mischung wurde 1 Std. gerührt und dann auf Eiswasser gegossen. Die organische Phase wurde abgetrennt und die wässrige Phase mit Dichlormethan (2 × 50 ml) nachextrahiert. Die vereinigten organischen Phasen wurden mit Wasser gewaschen und über Magnesiumsulfat getrocknet. Das nach Abdampfen erhaltene Rohprodukt wurde aus Hexan bei 0 °C umkristallisiert. Es wurden 8,5 g (98 % Ausbeute) reines Zielprodukt gewonnen.

(e) Herstellung von 1-[2-(trans-4-Pentylcyclohexyl)-1-ethyl]-4-(trans-4-propylcyclohexyl) methoxy-2-fluorbenzol

Eine Mischung aus dem nach Absatz (d) gewonnenen 3-Fluor-4-[2-(trans-4-pentylcyclohexyl)-1-ethyl] phenol (2,0 g, 0,00068 Mol), (trans-4-Propylcyclohexyl) methylbromid (2,0 g, 0,00089 Mol), wasserfreiem Kaliumcarbonat (3,8 g, 0,0274 Mol) und absolutem Dimethylformamid (100 ml) wurde 24 Std. unter Rückfluss erhitzt.

Das Reaktionsgemisch wurde wie in Absatz (d) aufgearbeitet. Das rohe Produkt wurde durch Säulenchromatographie an Silicagel mit Toluol als Eluiermittel gereinigt. Nach Abdampfen des Toluols wurde das Produkt mehrmals aus Ethanol umkristallisiert, bis die Phasenübergangstemperaturen konstant waren ; die entsprechenden Werte sind wie folgt : K 61,5 · N 110 · I.

Beispiele 2 bis 7

Nach dem in Beispiel 1 angegebenen Verfahren wurden unter Verwendung der entsprechenden (trans-4-Alkylcyclohexyl) methylbromide folgende erfindungsgemässen Verbindungen I hergestellt :

(Beispiel)

(2) 1-[2-(trans-4-Pentylcyclohexyl)-1-ethyl]-4-(trans-4-pentylcyclohexyl) methoxy-2-fluorbenzol ; K 72 · $S_A$ 73 · N 113 · I ;
(3) 1-[2-(trans-4-Pentylcyclohexyl)-1-ethyl]-4-(trans-4-heptylcyclohexyl) methoxy-2-fluorbenzol ; K 88 · $S_A$ 94 · N 110 · I ;
(4) 1-[2-(trans-4-Propylcyclohexyl)-1-ethyl]-4-(trans-4-propylcyclohexyl) methoxy-2-fluorbenzol ; K 64 · N 108 · I ;
(5) 1-[2-(trans-4-Propylcyclohexyl)-1-ethyl]-4-(trans-4-pentylcyclohexyl) methoxy-2-fluorbenzol ; K 54,5 · N 110,5 · I ;
(6) 1-[2-(trans-4-Propylcyclohexyl)-1-ethyl]-4-(trans-4-heptylcyclohexyl) methoxy-2-fluorbenzol ; K 72,5 · $S_A$ 76,5 · N 107,5 · I.
(7) 1-[2-(trans-4-Heptylcyclohexyl)-1-ethyl]-4-(trans-4-propylcyclohexyl) methoxy-2-fluorbenzol ; K 45 · N 108 I.

Beispiel 8

Aus 1-(4-trans-Heptylcyclohexyl)-2-(3-fluor-4-hydroxyphenyl)-ethan [F. 70-71° ; erhältlich analog Beispiel 1] und (trans-4-Propylcyclohexyl) methylbromid erhält man analog Beispiel 1 1-[2-(trans-4-Heptylcyclohexyl)-1-ethyl]-4-(trans-4-propyl-cyclohexyl) methoxy-3-fluorbenzol ; K 50,5 · $S_A$ 90 · N 110 · I.

Beispiel 9

Aus 1-(4-trans-Heptylcyclohexyl)-2-(3-brom-4-hydroxyphenyl)-ethan [F. 50-51° ; erhältlich durch Bromierung von 1-(4-trans-Heptylcyclohexyl)-2-(4-hydroxyphenyl)-ethan in Dichlormethan] erhält man analog Beispiel 8 1-[2-trans-4-Heptylcyclohexyl)-1-ethyl]-4-(trans-4-propylcyclohexyl) methoxy-3-brombenzol ; K 44 · N 85,5 · I.

Beispiel 10

Aus 1-(4-trans-4-Heptylcyclohexyl)-2-(3-chlor-4-hydroxyphenyl)-ethan [F. 56-57°, erhältlich analog Beispiel 1] und (trans-4-Propylcyclohexyl) methylbromid erhält man analog Beispiel 1
1-[2-(trans-4-Heptylcyclohexyl)-1-ethyl]-4-(trans-4-propylcyclohexyl) methoxy-3-chlorbenzol ;
K 46 · N 96 · I.

Beispiel 11

Aus 1-(4-trans-4-Heptylcyclohexyl)-2-(3-cyano-4-hydroxyphenyl)-ethan und (trans-4-Propylcyclohexyl) methylbromid erhält man analog Beispiel 1
1-[2-(trans-4-Heptylcyclohexyl)-1-ethyl]-4-(trans-4-propylcyclohexyl) methoxy-3-cyanobenzol ;
K 57 · $S_A$ 90 · I.

Beispiel 12

Eine Mischung aus 1,0 g 1-(4-trans-Pentylcyclohexyl)-2-(2-fluor-4-hydroxyphenyl) ethan (Beispiel 1), 0,8 g 1-Brombutan, 1,9 g Kaliumcarbonat und 30 ml Butanon wird über Nacht gekocht. Das Reaktionsgemisch wird zu 250 ml Wasser gegeben und dreimal mit 50 ml Chloroform extrahiert. Die vereinigten organischen Phasen werden zweimal mit 500 ml Wasser gewaschen und über $MgSO_4$ getrocknet. Übliche Aufarbeitung liefert 1-(4-trans-Pentylcyclohexyl)-2-(2-fluor-4-butyloxyphenyl)-ethan ;
K 24 · N 26 · I.

Beispiele 13 bis 25

Nach dem in Beispiel 12 angegebenen Verfahren wurden unter Verwendung der entsprechenden 1-(4-trans-Alkylcyclohexyl)-2-(2-fluor-4-hydroxyphenyl)-ethane bzw. der entsprechenden 1-(4-trans-Alkylcyclohexyl)-2-(3-fluor-4-hydroxyphenyl)-ethane und der entsprechenden 1-Bromalkane folgende erfindungsgemäße Verbindungen I hergestellt :

(Beispiel)

(13) 1-(4-trans-Propylcyclohexyl)-2-(2-fluor-4-ethoxyphenyl)-ethan ; F. 22° ; K. 14° (monotrop) ;
(14) 1-(4-trans-Propylcyclohexyl)-2-(2-fluor-4-butoxyphenyl)-ethan ; F. 21° ; K. 12° (monotrop) ;
(15) 1-(4-trans-Propylcyclohexyl)-2-(2-fluor-4-hexyloxyphenyl)-ethan ; F. 28° ; K. 22° (monotrop) ;
(16) 1-(4-trans-Pentylcyclohexyl)-2-(2-fluor-4-ethoxyphenyl)-ethan ; F. 32° ; K. 28° (monotrop) ;
(17) 1-(4-trans-Pentylcyclohexyl)-2-(2-fluor-4-propoxyphenyl)-ethan ; F. 22,5° ; K. 10° (monotrop) ;
(18) 1-(4-trans-Pentylcyclohexyl)-2-(2-fluor-4-pentyloxyphenyl)-ethan ; F. 26° ; K. 12° (monotrop) ;
(19) 1-(4-trans-Pentylcyclohexyl)-2-(2-fluor-4-hexyloxyphenyl)-ethan ; F. 23,5° ; K. 29,5° ;
(20) 1-(4-trans-Heptylcyclohexyl)-2-(2-fluor-4-ethoxyphenyl)-ethan ; F. 46° ; K. 35° (monotrop) ;
(21) 1-(4-trans-Heptylcyclohexyl)-2-(2-fluor-4-butoxyphenyl)-ethan ; F. 31° ; K. 34° ;
(22) 1-(4-trans-Heptylcyclohexyl)-2-(2-fluor-4-hexyloxyphenyl)-ethan ; F. 29,5° ; K. 35° ;
(23) 1-(4-trans-Heptylcyclohexyl)-2-(3-fluor-4-methoxyphenyl)-ethan ; F. 32° ; K. 29° (monotrop) ;
(24) 1-(4-trans-Heptylcyclohexyl)-2-(3-fluor-4-ethoxyphenyl)-ethan ; F. 53° ; K. 34° (monotrop) ;
(25) 1-(4-trans-heptylcyclohexyl)-2-(3-fluor-4-hexyloxyphenyl)-ethan ; F. 45° ; 35° (monotrop).

Beispiel 26

Ein Gemisch von 0,65 g trans-4-Pentylcyclohexan-carbonsäurechlorid und 10 ml Toluol wird zu einem Gemisch aus 0,9 g 1-(trans-4-Pentylcyclohexyl)-2-(2-fluor-4-hydroxyphenyl)-ethan (Beispiel 1), 2 ml Pyridin und 10 ml Toluol gegeben und über Nacht gerührt. Das Reaktionsgemisch wird in kalte, verdünnte Salzsäure gegossen und wie üblich aufgearbeitet. Man erhält trans-4-Pentylcyclohexancarbonsäure 3-fluor-4-[2-(trans-4-Pentylcyclohexyl)-ethyl]-phenylester ; F. 72,5°, K. 147°.

Beispiele 27 bis 31

Nach dem in Beispiel 26 angegebenen Verfahren werden folgende erfindungsgemäße Verbindungen (I) hergestellt :

(27) trans-4-Propylcyclohexancarbonsäure 3-fluor-4-[2-(trans-4-propylcyclohexyl)-ethyl]-phenylester ; F. 72° ; K. 140° ;
(28) trans-4-Pentylcyclohexancarbonsäure 3-fluor-4-[2-(trans-4-propylcyclohexyl)-ethyl]-phenylester ;
F. 68° ; K. 146,5° ;
(29) trans-4-Heptylcyclohexancarbonsäure 3-fluor-4-[2-(trans-4-propylcyclohexyl)-ethyl]-phenylester ;
K. 84° · $S_A$ 91° · N 140° · I ;

(30) trans-4-Propylcyclohexancarbonsäure 3-fluor-4-[2-(trans-4-pentylcyclohexyl)-ethyl]-phenylester ; F. 65° ; K. 141° ;

(31) trans-4-Heptylcyclohexancarbonsäure 3-fluor-4-[2-(trans-4-pentylcyclohexyl)-ethyl]-phenylester ;

K. 100° · $S_A$ 111° · N 142° · I.

Weitere Beispiele für Verbindungen der Formel I sind die folgenden :

2-(trans-4-Pentyl-4'-bicyclohexyl)-1-(3-fluoro-4-pentylphenyl) ethan ;
2-(trans-4-Pentyl-4'-bicyclohexyl)-1-(2-fluoro-4-pentylphenyl) ethan ;
2-(trans-4-Pentyl-4'-bicyclohexyl)-1-(3-fluoro-4-butoxyphenyl) ethan ;
2-(trans-4-Pentyl-4'-bicyclohexyl)-1-(2-fluoro-4-butoxyphenyl) ethan ;
2-(trans-4-Pentyl-4'-bicyclohexyl)-1-(3-cyano-4-pentylphenyl) ethan ;
2-(trans-4-Pentyl-4'-bicyclohexyl)-1-(2-cyano-4-pentylphenyl) ethan ;
2-(trans-4-Pentyl-4'-bicyclohexyl)-1-(3-cyano-4-ethoxyphenyl) ethan ;
2-(trans-4-Pentyl-4'-bicyclohexyl)-1-(3-cyano-4-butoxyphenyl) ethan ;
2-(trans-4-Pentyl-4'-bicyclohexyl)-1-(3-cyano-4-hexyloxyphenyl) ethan ;
2-(trans-4-Pentyl-4'-bicyclohexyl)-1-(2-cyano-4-ethoxyphenyl) ethan ;
2-(trans-4-Pentyl-4'-bicyclohexyl)-1-(2-cyano-4-butoxyphenyl) ethan ;
2-(trans-4-Pentyl-4'-bicyclohexyl)-1-(2-cyano-4-hexyloxyphenyl) ethan ;
2-Fluoro-1-[2-(trans-4-propylcyclohexyl)-1-ethyl]-4-[2-(trans-4-pentylcyclohexyl)-1-ethyl] benzol ;
3-Fluoro-1-[2-(trans-4-propylcyclohexyl)-1-ethyl]-4-[2-(trans-4-pentylcyclohexyl)-1-ethyl] benzol ;
2-Fluoro-1,4-di-[2-(trans-4-pentylcyclohexyl)-1-ethyl] benzol ;
2,3-Difluoro-1,4-di-[2-(trans-4-propylcyclohexyl)-1-ethyl] benzol ;
2,3-Difluoro-1,4-di-[2-(trans-4-pentylcyclohexyl)-1-ethyl] benzol ;
2,3-Difluoro-1,4-di-[2-(trans-4-heptylcyclohexyl)-1-ethyl] benzol ;
2-Cyano-1-[2-(trans-4-propylcyclohexyl)-1-ethyl]-4-[2-(trans-4-pentylcyclohexyl)-1-ethyl] benzol ;
3-Cyano-1-[2-(trans-4-propylcyclohexyl)-1-ethyl]-4-[2-(trans-4-pentylcyclohexyl)-1-ethyl] benzol ;
2-Cyano-1,4-di-[2-(trans-4-pentylcyclohexyl)-1-ethyl] benzol ;
2,3-Dicyano-1,4-di-[2-(trans-4-propylcyclohexyl)-1-ethyl] benzol ;
2,3-Dicyano-1,4-di-[2-(trans-4-pentylcyclohexyl)-1-ethyl] benzol ;
2,3-Dicyano-1,4-di-[2-(trans-4-heptylcyclohexyl)-1-ethyl] benzol.

**Patentansprüche** (für die Vertragsstaaten : CH, DE, FR, GB, IT, LI, NL)

1. Nematische Verbindungen mit negativer DK-Anisotropie, gekennzeichnet durch die Formel I

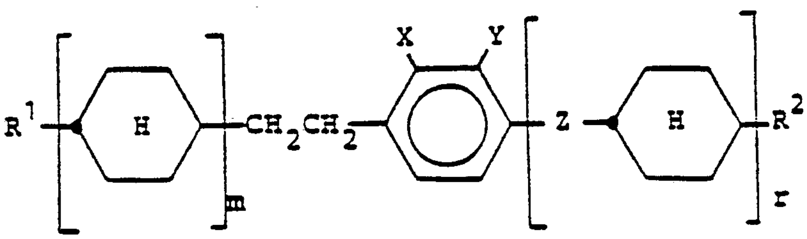

(I)

in welcher m = 1 oder 2, r = Null oder 1 und m + r = 1 oder 2 ist, und $R^1$ und $R^2$ gleich oder verschieden und gewählt sind aus Alkyl- und Alkoxygruppen mit jeweils 1-12 C-Atomen in gerader oder verzweigter und gegebenenfalls chiraler Kette, z ein Brückenglied gewählt aus der Einfachbindung und den Gruppen der Formeln $—CH_2CH_2—$, $—OCH_2—$, —O—CO— oder —CO—O— ist, eine der Gruppen X, Y gewählt ist aus Fluor, Chlor, Brom und Cyano und die andere der Gruppen X, Y gewählt ist aus Wasserstoff, Fluor, Chlor, Brom und Cyano, mit der Maßgabe, daß m = r = 1 ist, wenn beide X, Y Cyano bedeuten.

2. Verdindung nach Anspruch 1, dadurch gekennzeichnet, daß m + r = 2 ist.

3. Verbindung nach Anspruch 1, gekennzeichnet durch die Formel Ia

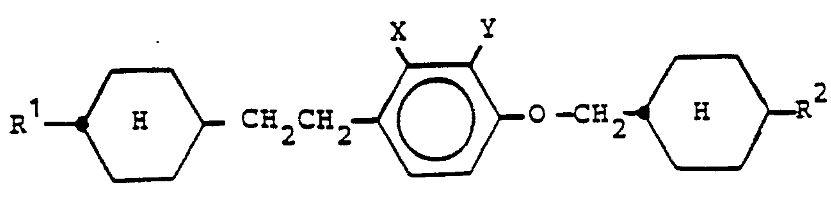

(Ia)

in der $R^1$, $R^2$, X und Y die in Anspruch 1 angegebene Bedeutung haben.

4. Verbindung nach Anspruch 1, gekennzeichnet durch die Formel Ib

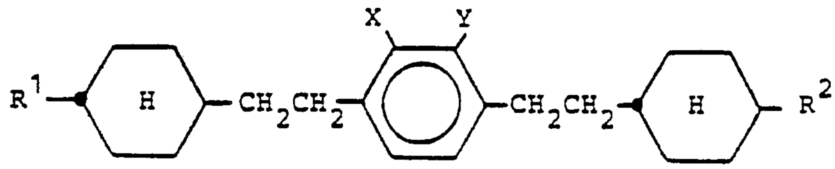

(Ib)

in der $R^1$, $R^2$, X und Y die in Anspruch 1 angegebene Bedeutung haben.

5. Verbindung nach Anspruch 1, gekennzeichnet durch die Formel Ic

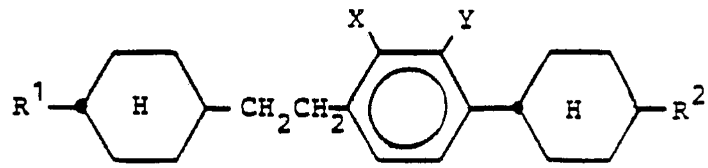

(Ic)

in der $R^1$, $R^2$, X und Y die in Anspruch 1 angegebene Bedeutung haben.

6. Verbindung nach Anspruch 1, gekennzeichnet durch die Formel Id

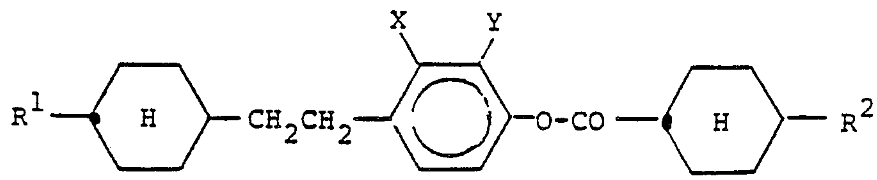

(Id)

in der $R^1$, $R^2$, X und Y die in Anspruch 1 angegebene Bedeutung haben.

7. Verbindung nach Anspruch 1, gekennzeichnet durch die Formel Ie

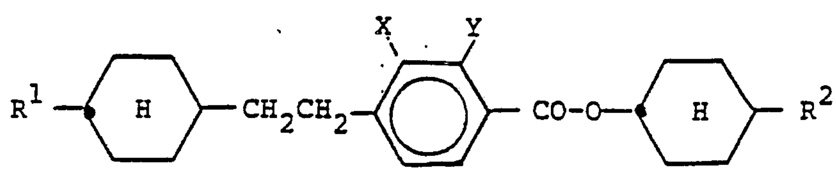

(Ie)

in der $R^1$, $R^2$, X und Y die in Aknspruch 1 angegebene Bedeutung haben.

8. Verbindung nach Anspruch 1, gekennzeichnet durch die Formel If

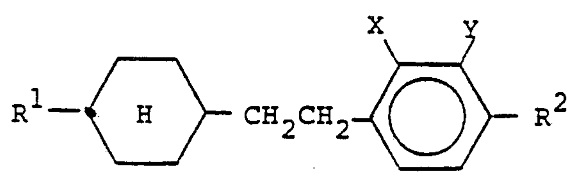

(If)

in der $R^1$, $R^2$, X und Y die in Anspruch 1 angegebene Bedeutung haben.

9. Flüssigkristallmischung, dadurch gekennzeichnet, daß sie mindestens eine Verbindung der Formel I gemäß Anspruch 1 als Komponente enthält.

10. Mischung nach Anspruch 9, dadurch gekennzeichnet, daß sie mindestens eine Verbindung der Formel I enthält, in der $m + r = 2$ ist.

11. Mischung nach Anspruch 9, dadurch gekennzeichnet, daß sie mindestens eine Verbindung der Formeln Ia, Ib, Ic, Id, Ie oder If gemäß den Ansprüchen 3, 4, 5, 6, 7 oder 8 enthält.

12. Verwendung der Verbindungen der Formel I nach Anspruch 1 als Komponenten von Flüssigkristallmischungen.

13. Elektrooptisches Anzeigeelement, dadurch gekennzeichnet, daß es eine Mischung nach einem der Ansprüche 9, 10 oder 11 enthält.

**Patentansprüche** (für den Vertragsstaat AT)

1. Flüssigkristallmischung, dadurch gekennzeichnet, daß sie mindestens eine nematische Verbindung mit negativer DK-Anisotropie enthält, gekennzeichnet durch die Formel I

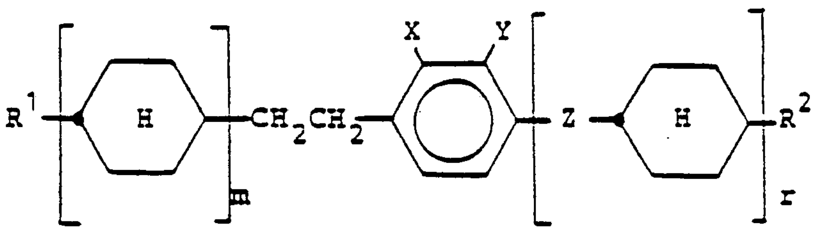

(I)

in welcher m = 1 oder 2, r = Null oder 1 und m + r = 1 oder 2 ist, und $R^1$ und $R^2$ gleich oder verschieden und gewählt sind aus Alkyl- und Alkoxygruppen mit jeweils 1-12 C-Atomen in gerader oder verzweigter und gegebenenfalls chiraler Kette, z ein Brückenglied gewählt aus der Einfachbindung und den Gruppen der Formeln $—CH_2CH_2—$, $—OCH_2—$, —O—CO— oder —CO—O— ist, eine der Gruppen X, Y gewählt ist aus Fluor, Chlor, Brom und Cyano und die andere der Gruppen X, Y gewählt ist aus Wasserstoff, Fluor, Chlor, Brom und Cyano, mit der Maßgabe, daß m = r = 1 ist, wenn beide X, Y Cyano bedeuten.

2. Flüssigkristallmischung nach Anspruch 1, dadurch gekennzeichnet, daß m + r = 2 ist.

3. Flüssigkristallmischung nach Anspruch 1, dadurch gekennzeichnet, daß sie mindestens eine Verbindung der Formel Ia enthält,

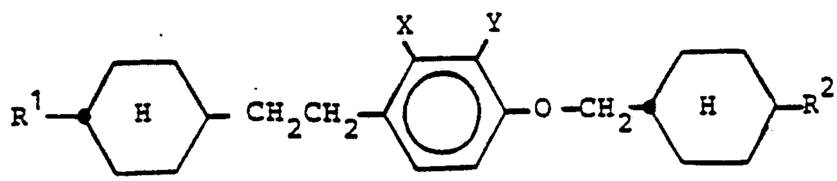

(Ia)

in der $R^1$, $R^2$, X und Y die in Anspruch 1 angegebene Bedeutung haben.

4. Flüssigkristallmischung nach Anspruch 1, dadurch gekennzeichnet, daß sie mindestens eine Verbindung der Formel Ib enthält,

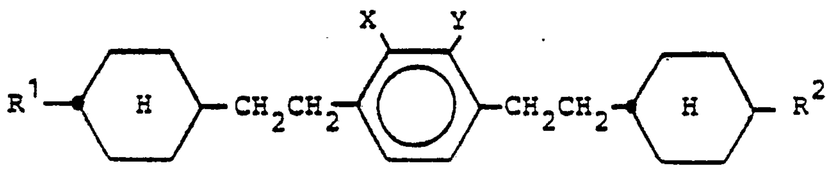

(Ib)

in der $R^1$, $R^2$, X und Y die in Anspruch 1 angegebene Bedeutung haben.

5. Flüssigkristallmischung nach Anspruch 1, dadurch gekennzeichnet, daß sie mindestens eine Verbindung der Formel Ic enthält,

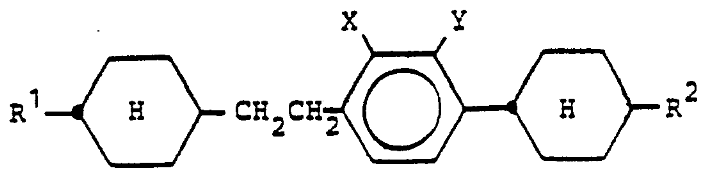

(Ic)

in der $R^1$, $R^2$, X und Y die in Anspruch 1 angegebene Bedeutung haben.

6. Flüssigkristallmischung nach Anspruch 1, dadurch gekennzeichnet, daß sie mindestens eine Verbindung der Formel Id enthält,

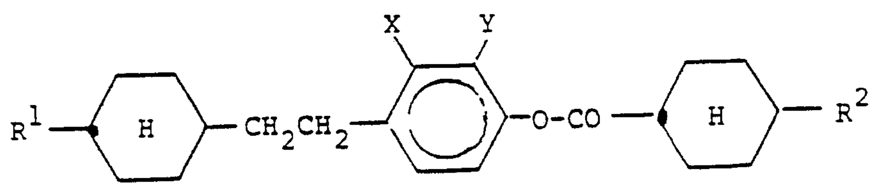

(Id)

in der $R^1$, $R^2$, X und Y die in Anspruch 1 angegebene Bedeutung haben.

7. Flüssigkristallmischung nach Anspruch 1, dadurch gekennzeichnet, daß sie mindestens eine Verbindung der Formel Ie enthält,

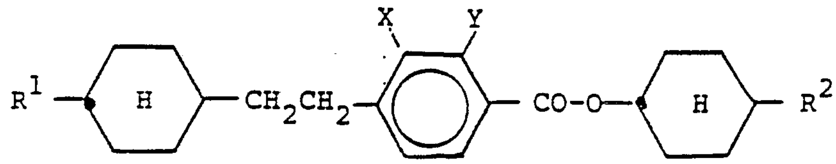

(Ie)

in der $R^1$, $R^2$, X und Y die in Anspruch 1 angegebene Bedeutung haben.

8. Flüssigkristallmischung nach Anspruch 1, dadurch gekennzeichnet, daß sie mindestens eine Verbindung der Formel If enthält,

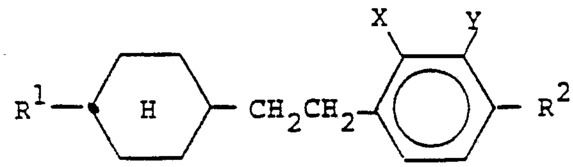

(If)

in der $R^1$, $R^2$, X und Y die in Anspruch 1 angegebene Bedeutung haben.

9. Verwendung der Verbindungen der Formel I nach Anspruch 1 als Komponenten von Flüssigkristallmischungen.

10. Elektrooptisches Anzeigeelement, dadurch gekennzeichnet, daß es eine Mischung nach einem der Ansprüche 1 bis 8 enthält.

**Claims** (for the Contracting States : CH, DE, FR, GB, IT, LI, NL)

1. Nematic compounds with a negative anisotropy of the dielectric constant, characterised by the formula (I)

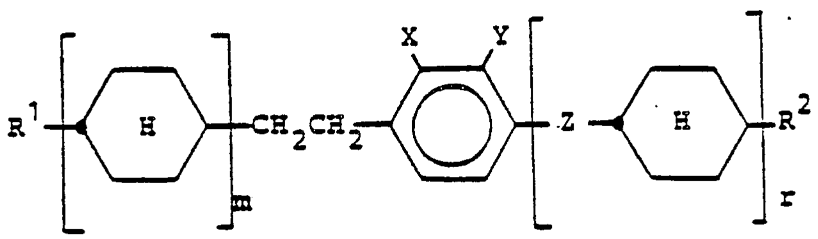

(I)

in which m = 1 or 2, r is zero or 1 and m + r = 1 or 2, and $R^1$ and $R^2$ are identical or different and are selected from alkyl and alkoxy groups each having 1-12 carbon atoms in a straight or branched chain, which may be chiral, Z is a bridge member selected from a single bond and the groups of the formulae $—CH_2CH_2—$, $—OCH_2—$, —O—CO— or —CO—O—, one of the groups X and Y is selected from fluorine, chlorine, bromine and cyano and the other of the groups X and Y is selected from hydrogen, fluorine, chlorine, bromine and cyano, with the proviso that m = r = 1 if X and Y are both cyano.

2. Compound according to Claim 1, characterised in that m + r = 2.

3. Compound according to Claim 1, characterised by the formula Ia

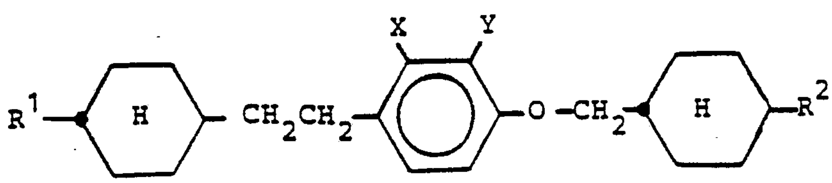

(Ia)

in which $R^1$, $R^2$, X and Y have the meanings given in Claim 1.

4. Compound according to Claim 1, characterised by the formula Ib

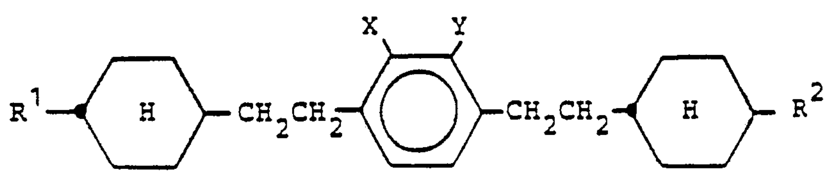

(Ib)

in which $R^1$, $R^2$, X and Y have the meanings given in Claim 1.

5. Compound according to Claim 1, characterised by the formula Ic

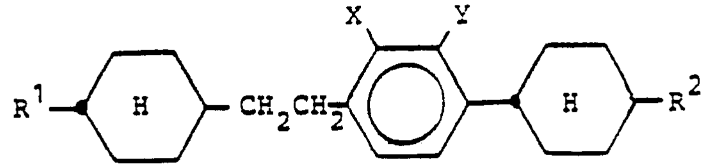

(Ic)

in which $R^1$, $R^2$, X and Y have the meanings given in Claim 1.

6. Compound according to Claim 1, characterised by the formula Id

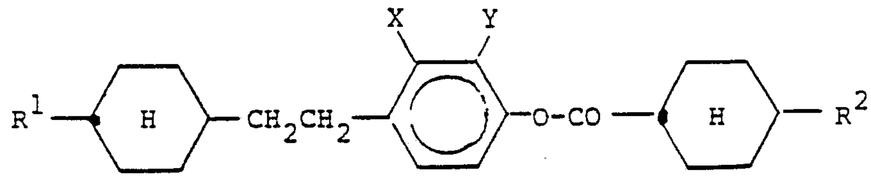

(Id)

in which $R^1$, $R^2$, X and Y have the meanings given in Claim 1.

7. Compound according to Claim 1, characterised by the formula Ie

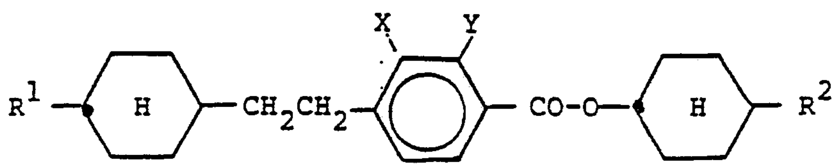

(Ie)

in which $R^1$, $R^2$, X and Y have the meanings given in Claim 1.

8. Compound according to Claim 1, characterised by the formula If

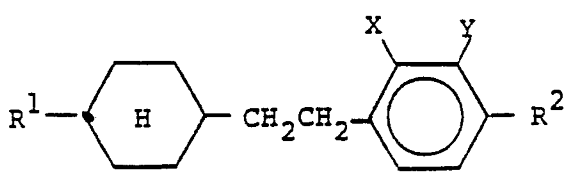

(If)

in which $R^1$, $R^2$, X and Y have the meanings given in Claim 1.

9. Liquid crystal mixture, characterised in that it contains at least one compound of the formula (I) according to Claim 1, as a component.

10. Mixture according to Claim 9, characterised in that in contains at least one compound of the formula (I) in which $m + r = 2$.

11. Mixture according to Claim 9, characterised in that it contains at least one compound of the formulae Ia, Ib, Ic, Id, Ie, or If according to Claims 3, 4, 5, 6, 7 or 8.

12. Use of the compounds of the formula (I) according to Claim 1, as components of liquid crystal mixtures.

13. Electro-optical display element, characterised in that it contains a mixture according to one of Claims 9, 10 or 11.

**Claims** (for the Contracting State AT)

1. Liquid crystal mixture, characterised in that it contains at least one nematic compound with a negative anisotropy of the dielectric constant, characterised by the formula (I)

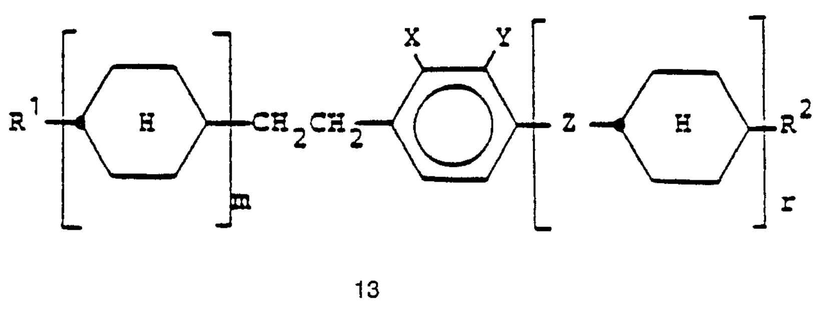

(I)

in which m = 1 or 2, r is zero or 1 and m + r = 1 or 2, and $R^1$ and $R^2$ are identical or different and are selected from alkyl and alkoxy groups each having 1-12 carbon atoms in a straight or branched chain, which may be chiral, Z is a bridge member selected from a single bond and the groups of the formulae $-CH_2CH_2-$, $-OCH_2-$, —O—CO— or —CO—C—, one of the groups X and Y is selected from fluorine, chlorine, bromine and cyano and the other of the groups X and Y is selected from hydrogen, fluorine, chlorine, bromine and cyano, with the proviso that m = r = 1 if X and Y are both cyano.

2. Liquid crystal mixture according to Claim 1, characterised in that m + r = 2.

3. Liquid crystal mixture according to Claim 1, characterised in that it contains at least one compound of the formule Ia.

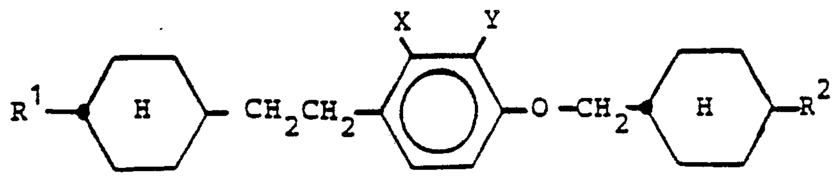
(Ia)

in which $R^1$, R*2, X and Y have the meanings given in Claim 1.

4. Liquid crystal mixture according to Claim 1, characterised in that it contains at least one compound of the formula Ib

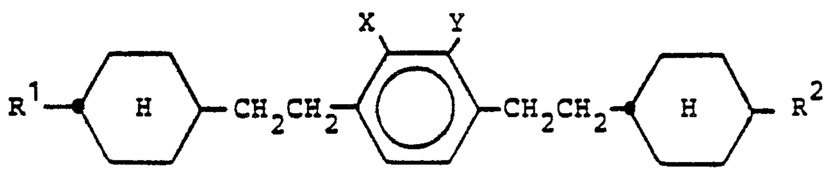
(Ib)

in which $R^1$, R*2, X and Y have the meanings give in Claim 1.

5. Liquid crystal mixture according to Claim 1, characterised in that it contains at least one compound of the formula Ic

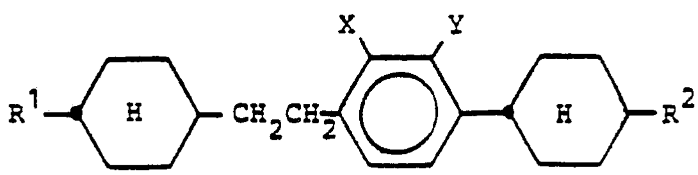
(Ic)

in which $R^1$, $R^2$, X and Y have the meanings given in Claim 1.

6. Liquid crystal mixture according to Claim 1, characterised in that it contains at least one compound of the formula Id

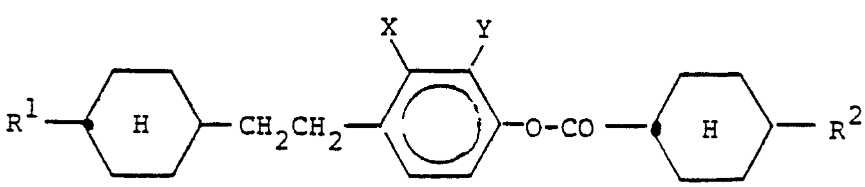
(Id)

in which $R^1$, $R^2$, X and Y have the meanings given in Claim 1.

7. Liquid crystal mixture according to Claim 1, characterised in that it contains at least one compound of the formula Ie

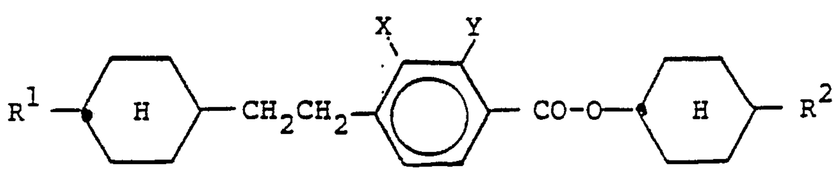
(Ie)

in which $R^1$, $R^2$, X and Y have the meanings given in Claim 1.

8. Liquid crystal mixture according to Claim 1, characterised in that it contains at least one compound of the formula If

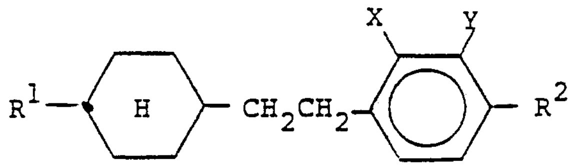

(If)

in which $R^1$, $R^2$, X and Y have the meanings given in Claim 1.

9. Use of the compounds of the formula (I) according to Claim 1, as components of liquid crystal mixtures.

10. Electro-optical display element, characterised in that it contains a mixture according to one of Claims 1 to 8.

**Revendications** (pour les Etats contractants : CH, DE, FR, GB, IT, LI, NL)

1. Composés nématiques à anisotropie diélectrique négative, caractérisés par la formule I

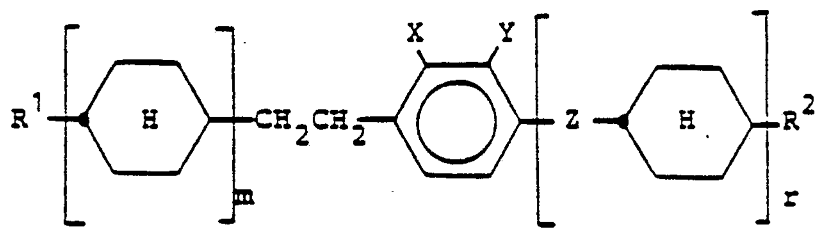

(I)

dans laquelle m = 1 ou 2, r = 0 ou 1 et m + r = 1 ou 2 et $R^1$ et $R^2$, ayant des significations identiques ou différentes, sont choisis parmi les groupes alkyle et alcoxy contenant chacun 1 à 12 atomes de carbone en chaîne droite ou ramifiée et le cas échéant chirale, Z représente un pont choisi parmi les liaisons simples et les groupes de formules $—CH_2CH_2—$, $—OCH_2—$, —O—CO— ou —CO—O—, l'un des substituants X et Y est choisi parmi le fluor, le chlore, le brome et le cyano et l'autre parmi l'hydrogène, le fluor, le chlore, le brome et le cyano, avec la condition supplémentaire que m = r = 1 lorsque les deux symboles X et Y représentent des groupes cyano.

2. Composé selon la revendication 1, caractérisé en ce que m + r = 2.

3. Composé selon la revendication 1, caractérisé par la formule Ia

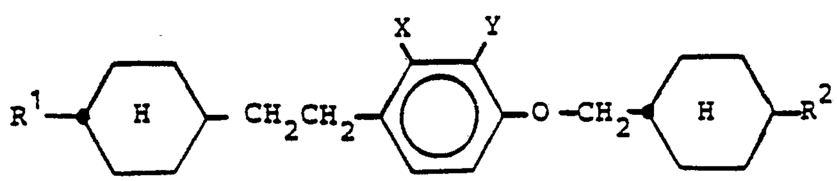

(Ia)

dans laquelle $R^1$, $R^2$, X et Y ont les significations indiquées dans la revendication 1.

4. Composé selon la revendication 1, caractérisé par la formule Ib

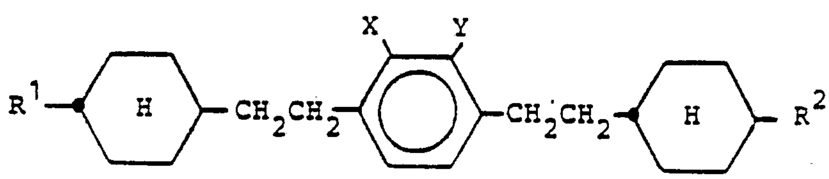

(Ib)

dans laquelle $R^1$, $R^2$, X et Y ont les significations indiquées dans la revendication 1.

5. Composé selon la revendication 1, caractérisé par la formule Ic.

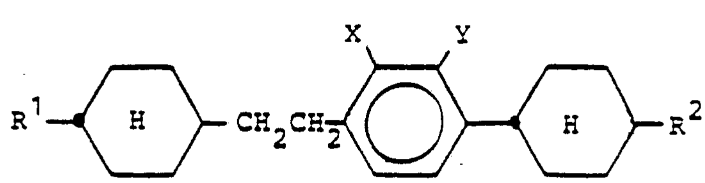

(Ic)

dans laquelle $R^1$, $R^2$, X et Y ont les significations indiquées dans la revendication 1.

6. Composé selon la revendication 1, caractérisé par la formule Id

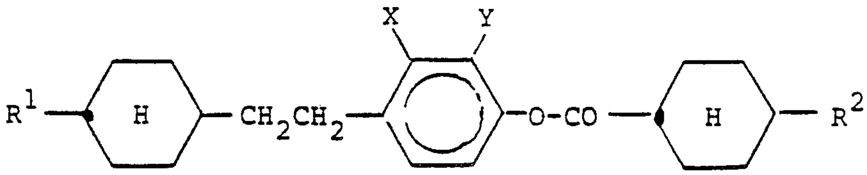

(Id)

dans laquelle $R^1$, $R^2$, X et Y ont les significations indiquées dans la revendication 1.

7. Composé selon la revendication 1, caractérisé par la formule Ie

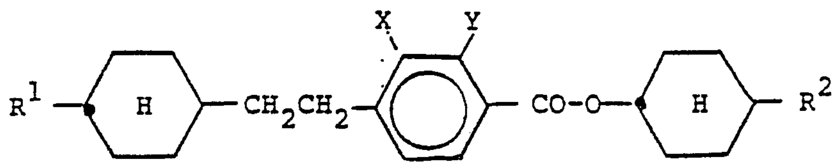

(Ie)

dans laquelle $R^1$, $R^2$, X et Y ont les significations indiquées dans la revendication 1.

8. Composé selon la revendication 1, caractérisé par la formule If

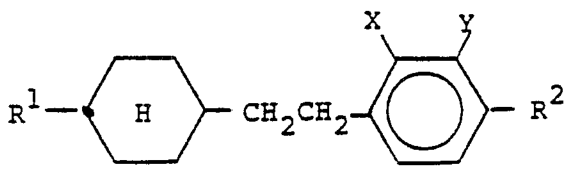

(If)

dans laquelle $R^1$, $R^2$, X et Y ont les significations indiquées dans la revendication 1.

9. Mélange à cristaux liquides, caractérisé en ce qu'il contient au moins un composé de formule I selon la revendication 1 en tant que composant.

10. Mélange selon la revendication 9, caractérisé en ce qu'il contient au moins un composé de formule I dans laquelle m + r = 2.

11. Mélange selon la revendication 9, caractérisé en ce qu'il contient au moins un composé de formule Ia, Ib, Ic, Id, Ie, ou If selon les revendications 3, 4, 5, 6, 7 ou 8.

12. Utilisation des composés de formule I selon la revendication 1 en tant que composants de mélanges à cristaux liquides.

13. Elément d'affichage électro-optique, caractérisé en ce qu'il contient un mélange selon l'une des revendications 9, 10 ou 11.

**Revendications** (pour l'Etat contractant AT)

1. Mélange à cristaux liquides, caractérisé en ce qu'il contient au moins un composé nématique à anisotropie diélectrique négative caractérisé par la formule I

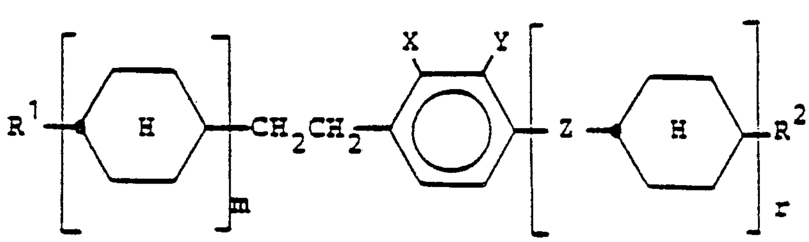

(I)

dans laquelle m = 1 ou 2, r = 0 ou 1 et m + r = 1 ou 2 et $R^1$ et $R^2$, ayant des significations identiques ou différentes, sont choisis parmi les groupes alkyle et alcoxy contenant chacun 1 à 12 atomes de carbone en chaîne droite ou ramifiée et le cas échéant chirale, Z représente un pont choisi parmi les liaisons simples et les groupes de formule $-CH_2CH_2-$, $-OCH_2-$, —O—CO— ou —CO—O—, l'un des substituants X et Y est choisi parmi le fluor, le chlore, le brome et le cyano et l'autre parmi l'hydrogène, le fluor, le chlore, le brome et le cyano, avec la condition supplémentaire que m = r = 1 lorsque les deux symboles X et Y représentent des groupes cyano.

2. Mélange à cristaux liquides selon la revendication 1, caractérisé en ce que m + r = 2.

3. Mélange à cristaux liquide selon la revendication 1, caractérisé en ce qu'il contient au moins un composé de formule Ia

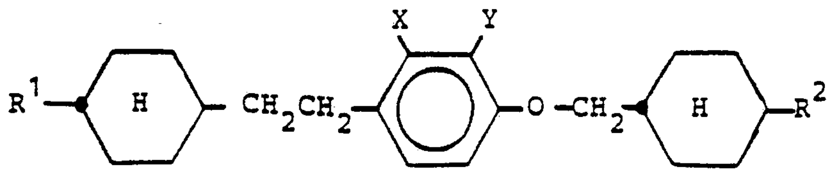

(Ia)

dans laquelle $R^1$, $R^2$, X et Y ont les significations indiquées dans la revendication 1.

4. Mélange à cristaux liquides selon la revendication 1, caractérisé en ce qu'il contient au moins un composé de formule Ib

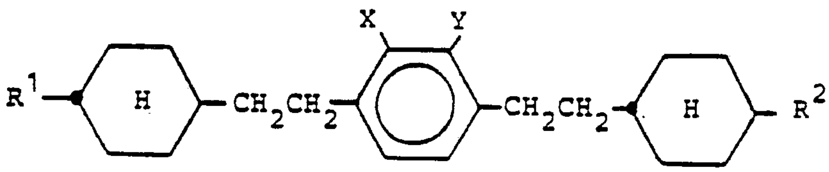

(Ib)

dans laquelle $R^1$, $R^2$, X et Y ont les significations indiquées dans la revendication 1.

5. Mélange à cristaux liquides selon la revendication 1, caractérisé en ce qu'il contient au moins un composé de formule Ic

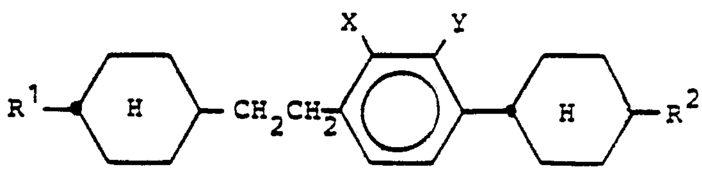

(Ic)

dans laquelle $R^1$, $R^2$, X et Y ont les significations indiquées dans la revendication 1.

6. Mélange à cristaux liquides selon la revendication 1, caractérisé en ce qu'il contient au moins un composé de formule Id

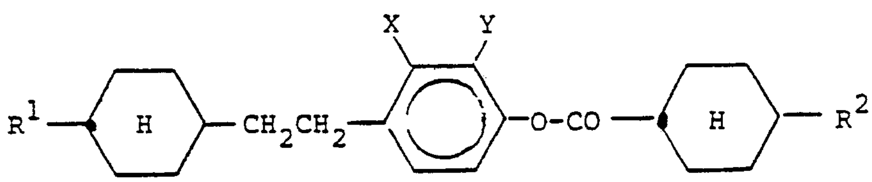

(Id)

dans laquelle $R^1$, $R^2$, X et Y ont les significations indiquées dans la revendication 1.

7. Mélange à cristaux liquides selon la revendication 1, caractérisé en ce qu'il contient au moins un composé de formule Ie

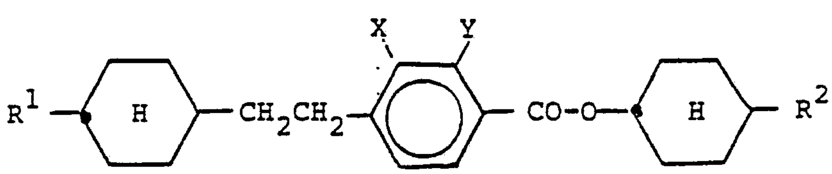

(Ie)

dans laquelle $R^1$, $R^2$, X et Y ont les significations indiquées dans la revendication 1.

8. Mélange à cristaux liquides selon la revendication 1, caractérisé en ce qu'il contient au moins un composé de formule If

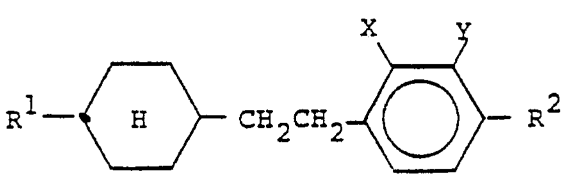

(If).

dans laquelle $R^1$, $R^2$, X et Y ont les significations indiquées dans la revendication 1.

9. Utilisation des composés de formule I selon la revendication 1 en tant que composants de mélanges à cristaux liquides.

10. Elément d'affichage électro-optique, caractérisé en ce qu'il contient un mélange selon l'une des revendications 1 à 8.